# EUROPEAN PATENT APPLICATION

(11) **EP 1 645 284 A1**
(43) Date of publication of application: **12.04.2006**
(21) Application number: 04024106.9
(22) Date of filing: 08.10.2004
(51) Int. Cl.: A61K 39/395, C07K 16/28, A61P 31/04, A61P 35/00, G01N 33/53

(54) **Use of ligands of the antigen CDw52 for the treatment of diseases that are related to degenerated macrophages**

(71) Applicant: Charité - Universitätsmedizin Berlin, 10117 Berlin (DE)
(72) Inventor: Gaedicke, Gerhard, 10829 Berlin (DE); Krenn, Veit, 13503 Berlin (DE); Günther, Raphaela, 10435 Berlin (DE); Lode, Holger, 13469 Berlin (DE)
(74) Representative: Krauss, Jan

(57) **Abstract**

The present invention relates to the use of a ligand of CDw52 for the preparation of a medicament for the treatment of diseases that are related to CDw52 expressing degenerated macrophages. In particular, the present invention relates to the treatment of diseases such as MAS, LCH, HLH, and AML.

## Description

The present invention relates to the use of a ligand of CDw52 for the preparation of a medicament for the treatment of diseases that are related to CDw52 expressing degenerated macrophages. In particular, the present invention relates to the treatment of diseases such as MAS, LCH, HLH, and AML.

For the purposes of the present invention, all references as cited herein are incorporated by reference in their entireties.

### Description

Human CDw52 is a glycosylphosphatidylinositol (GPI)-anchored antigen expressed on the all lymphocytes as well as within the male genital tract, where it can be produced by epithelial cells of the distal epididymis and duct deferens. The physiological role of CDw52 on lymphocytes is unclear, although antibody directed to CDw52, CAMPATH-1, is capable of complement activation and it can be clinically useful to treat lympho-proliferative disorders and to deplete lymphocytes in bone marrow transplants (Domagala A, Kurpisz M. CD52 antigen - a review. Med Sci Monit. 2001 Mar-Apr;7(2):325-31).

Presently, CDw52-specific antibodies are used in B-cell chronic lymphocytic leukaemia (B-CLL) (Moreton P, Hillmen P. Alemtuzumab therapy in B-cell lymphoproliferative disorders. Semin Oncol. 2003 Aug;30(4):493-501; Lundin J, et al. Phase II trial of subcutaneous anti-CD52 monoclonal antibody alemtuzumab (Campath-1H) as first-line treatment for patients with B-cell chronic lymphocytic leukaemia (B-CLL). Blood. 2002 Aug 1;100(3):768-73.). The efficiency of the therapy correlates with the quantity of the CDw52 expression (D'Arena G, et al. Quantitative evaluation of CD52 expression in B-cell chronic lymphocytic leukaemia. Leuk Lymphoma. 2003 Jul;44(7):1255-7.). The CDw52 antibody is furthermore used in the therapy of transplantation rejection (Hale G, et al. CD52 antibodies for prevention of graft-versus-host disease and graft rejection following transplantation of allogeneic peripheral blood stem cells. Bone Marrow Transplant. 2000 Jul;26(1):69-76.) and, most recently, in Behcet's disease (Lockwood CM, Hale G, Waldman H, Jayne DR. Remission induction in Behcet's disease following lymphocyte depletion by the anti-CD52 antibody CAMPATH 1-H. Rheumatology (Oxford). 2003 Aug 29).

Alemtuzumab (Campath-1H, Ilex Pharmaceuticals, San Antonio, TX, USA) is a humanised monoclonal antibody that recognises the CDw52 antigen expressed on malignant and normal B lymphocytes. It has come to be used therapeutically in B-cell malignancies. Responses are seen in non-Hodgkin's lymphoma (NHL), and alemtuzumab can induce molecular remissions in relapsed chronic lymphocytic leukaemia (CLL), even when refractory to purine analogues. Most studies reveal the responses to be superior in the absence of bulky disease. Infusion-related side effects such as rigors, hypotension, and nausea are reduced by using the subcutaneous route of administration. Infectious complications are the most important toxicity seen and are related to the depletion of normal lymphocytes. The clinical efficacy in combination with both fludarabine and rituximab is under investigation.

Alemtuzumab is an unconjugated, humanised, monoclonal antibody directed against the cell surface antigen CDw52 on lymphocytes and monocytes. In noncomparative phase I/II studies in patients with B-cell chronic lymphocytic leukaemia (B-CLL) relapsed after or refractory to alkylating agents and fludarabine, intravenous (IV) administration of alemtuzumab 30 mg/day three times weekly for up to 12 weeks was associated with overall objective response (OR) rates of 21-59% (Frampton JE, Wagstaff AJ. Alemtuzumab. Drugs. 2003;63(12):1229-43; discussion 1245-6).

The biological effect of ligands of CDw52 seems to be mediated by an unusual lytic effect on the target cells (Kirk AD, et al. Results from a human renal allograft tolerance trial evaluating the humanised CD52-specific monoclonal antibody alemtuzumab (CAMPATH-1H). Transplantation. 2003 Jul 15;76(1):120-9).

CA 2,060,384 describes a method of treatment of a human or animal subject suffering from T-cell mediated inflammation of the joints, such as rheumatoid arthritis, which comprises administering to said subject an effective amount of an antibody recognising the CDw52 antigen. Preferably the antibody is CAMPATH-1H administered in an amount of 1 to about 100mg per day for a period between 1 and 30 days.

WO 93/01296 describes a recombinant vaccinia virus capable of expressing on infection of a suitable cell the light chain and/or the heavy chain of an antibody and the use of such a virus in the production of recombinant antibodies. The antibody may be a chimeric or a humanised (CDR-grafted) antibody for example a humanised antibody against the CDw52 antigen.

WO 93/08837 describes a stabilised immunoglobulin composition comprising at least one immunoglobulin together with a stabilising amount of a chelator of copper ions such as EDTA or citrate. Preferably the immunoglobulin is an antibody, for example a recombinant CDR-grafted antibody against the CDw52 antigen, most preferably CAMPATH-1H.

CH 681305 describes a purified preparation of an anti-CDw52 antibody which exhibits on size exclusion chromatography: a single peak under non-reducing conditions and two major peaks under reducing conditions. Also a process of purifying an anti-CDw52 antibody, formulations containing such a purified preparation and uses thereof are described.

US 5,786,176 and WO 94/02604 describe recombinant cell lines, in particular mammalian cell lines, capable of expressing recombinant CDw52 antigen or an antigenic fragment thereof. The cell lines can be used for the production of recombinant CDw52 antigen and in assaying for anti-CDw52 antibody in a sample.

US 6,120,766 and WO 93/10817 describe the use of an antibody recognising the CDw52 antigen in the treatment of multiple sclerosis. Preferably, the antibody is the humanised antibody in the humanised antibody CAMPATH-1H.

WO 93/07899 describes the use of an antibody recognising the CDw52 antigen for the treatment of T-cell mediated inflammation of the joints, in particular for the treatment of rheumatoid arthritis. Preferably the antibody is the humanised antibody CAMPATH-1H which may be used together with another immunosuppressive antibody or an immunosuppressive agent such as a steroid.

Macrophage activation syndrome (MAS) is a serious complication of childhood systemic inflammatory disorders that is thought to be caused by excessive activation and proliferation of T lymphocytes and macrophages. Recent findings in hemophagocytic lymphohistiocytosis HLH), a disease that is clinically similar to MAS, highlight the possible pathogenetic role of a defective function of perforin, a protein involved in the cytolytic processes and control of lymphocyte proliferation. Reactive macrophage activation syndrome or haemophagocytic lymphohistiocytosis, by the activation of the mononuclear phagocytic system with prominent haemophagocytosis particularly involving the bone marrow, reticuloendothelial system and central nervous system, is a life-threatening disorder. It frequently occurs in children as a familial autosomal recessive disorder and it has been reportedly associated with infections, underlying hematopoetic malignancies or autoimmune disorders. It has been suggested that central to the pathogenesis of this disorder is uncontrolled T-cell activation which results in the excessive secretion of cytokines. The clinical and laboratory findings are dramatic, at presentation with persistent fever, hepatosplenomegaly, profound depression of three blood cell lines, elevated serum liver enzyme values, coagulopathy and abundant haemophagocytosis can be visualised in bone marrow. Most of the described clinical findings could be explained by the oversecretion of proinflammatory cytokines and the presence of histiocytic infiltrates. In general, immunosuppressive therapy, cytotoxic chemotherapy with immunomodulatory agents will induce remissions in a substantial percentage of patients, whereas others require allogenic bone marrow transplantation. Cyclosporin A has been found effective in patients with corticosteroid-resistant MAS. A recent report has suggested that etanercept may be a useful adjunctive therapeutic agent. (Szamosi S, Szucs G, Zeher M, Szegedi G. Reactive macrophage activation syndrome Orv Hetil. 2003 Sep 14;144(37):1803-8; Ravelli A. Macrophage activation syndrome. Curr Opin Rheumatol. 2002 Sep;14(5):548-52.). See also Imashuku (Shinsaku Imashuku, Advances in the management of haemophagocytic lymphohistiocytosis, Int. J. Hematology, 2000, pages 1-11, and Imashuku S, Teramura T, Morimoto A, Hibi S. Recent developments in the management of haemophagocytic lymphohistiocytosis. Expert Opin Pharmacother. 2001 Sep;2(9):1437-48.).

Langerhans cell histiocytosis (LCH) is a group of idiopathic disorders characterised by the proliferation of specialised bone marrow-derived Langerhans cells (LCs) and mature eosinophils. The term LCH is generally preferred to the older term, histiocytosis X. The working group of the Histiocyte Society has divided histocytic disorders into 3 different groups: (1) dendritic cell histiocytosis, (2) erythrophagocytic macrophage disorders, and (3) malignant histiocytosis. LCH belongs in group 1 and encompasses a number of diseases. The clinical spectrum includes on one end, an acute fulminant, disseminated disease called Letterer-Siwe disease and, on the other end, solitary or few, indolent and chronic, lesions of bone or other organs called eosinophilic granulomas. The intermediate clinical form called Hand-Schuller-Christian disease is characterised by multifocal, chronic involvement and classically presents as the triad of diabetes insipidus, proptosis, and lytic bone lesions. A congenital self-healing form called Hashimoto-Pritzker disease has also been described.

The pathogenesis of LCH is unknown. An ongoing debate exists over whether this is a reactive or neoplastic process. Arguments supporting the reactive nature of this disorder include the occurrence of spontaneous remissions, the failure to detect aneuploidy, metaphase or karyotypic abnormalities, and the good survival rate in patients without organ dysfunction. On the other hand, the infiltration of organs by aberrant cells, a possible lethal evolution, and the cancer-based modalities of successful treatment are all consistent with a neoplastic process. In addition, the demonstration of LCH as a monoclonal proliferation by X chromosome-linked DNA probes supports a neoplastic origin for this proliferation; however, the presence of this finding in distinct subtypes with different evolutions demands further investigations to evaluate its significance. In the US, LCH is a rare disease. The estimated annual incidence ranges from 0.5-5.4 per million persons per year. Approximately 1200 new cases per year are reported in the United States. As a therapy for LCH, bisphosphonates as antialveolar macrophage therapy have been proposed (Brown RE. Bisphosphonates as antialveolar macrophage therapy in pulmonary langerhans cell histiocytosis? Med Pediatr Oncol. 2001 Jun;36(6):641-3.).

Acute Myelogenous Leukemia (AML) is the accumulation of immature myeloid cells, as compared to myelodyplastic syndromes and myeloproliferative syndromes. AML comprises about 15% of childhood leukemia in the US, although it accounts for the majority of congenital leukemia. Traditionally, AML is defined by the presence of more than 30% myeloid blasts in the marrow, although it occassionally manifests with extramedullary disease followed by later marrow findings.

In AML, at least one of the following cell surface determinants is usually positive: CD 11b, 13, 14, 15, 33, 36. Cytogenetic abnormalities are present in almost all cases of AML.

The presenting signs of AML usually relate to bone marrow replacement, anaemia, and accumulation of cells in the reticuloendothelial system. Bone marrow failure and infiltration can result in variable anemia, granulocytopenia, and thrombocytopenia. In about half of the cases, hepatosplenomegaly is present, and lymphadenopathy may also occur. The long term event free survival of AML patients in general is about 3-45%. Poor prognostic factors include high white counts, the presence of monosomy 7, the development of AML after a myelodysplastic syndrome or chemotherapy treatment, and the presence of the multidrug resistance glycoprotein. Good prognosis is associated with the t(8;21) and inv(16) karyotypes associated with M4Eo, and with t(15;17) and t(9;11).

The initial treatment of AML may involve stabilisation: correction of coagulopathy, treatment of hyperviscosity (by leukophoresis, hydroxyurea, exchange transfusion), and correction of electrolyte disturbances. Patients are then placed on hydration and allopurinol to prevent tumour lysis syndrome and started on chemotherapy as quickly as possible. Treatment of AML commonly uses multidrug regimens which are more toxic and myeloablative than the ALL protocols. About 75-80% of patients achieve a complete remission (no peripheral blasts and marrow < 5% blasts). Toxic deaths in the induction period range 5-10%. The patients then proceed to an intensification regimen, without which 90% would spontaneously relapse. When a matched sibling is available, allogeneic stem cell transplant is preferable. Unmatched transplants may be considered for treatment-related AML and in cases of monosomy 7 where the natural history of the disease is worse.

Further references that are directed to markers of macrophages are, for example Buggins, et al. (Buggins AG, Mufti GJ, Salisbury J, Codd J, Westwood N, Arno M, Fishlock K, Pagliuca A, Devereux S. Peripheral blood but not tissue dendritic cells express CD52 and are depleted by treatment with alemtuzumab. Blood. 2002 Sep 1;100(5):1715-20.), Taylor et al. (Taylor ML, Noble PW, White B, Wise R, Liu MC, Bochner BS. Extensive surface phenotyping of alveolar macrophages in interstitial lung disease. Clin Immunol. 2000 Jan;94(1):33-41.), Lichtenberger et al. (Lichtenberger C, Zakeri S, Baier K, Willheim M, Holub M, Reinisch W. A novel high-purity isolation method for human peripheral blood neutrophils permitting polymerase chain reaction-based mRNA studies. J Immunol Methods. 1999 Jul 30;227(1-2):75-84.), Mumcuoglu and Slavin (Mumcuoglu M, Slavin S. Enhancement of hematopoietic reconstitution with recombinant cytokines: effect of rhIL-6 in combination with rhGM-CSF and rhIL-3 on unmodified or T cell-depleted bone marrow. J Hematother. 1999 Jun;8(3):247-53.), and Elsner et al. (Elsner J, Hochstetter R, Spiekermann K, Kapp A. Surface and mRNA expression of the CD52 antigen by human eosinophils but not by neutrophils. Blood. 1996 Dec 15;88(12):4684-93.)

For all of the above discussed diseases, alternative treatment options are needed. It is therefore an object of the present invention, to provide new treatment options for those macrophage-related diseases wherein said are degenerated (for example cancerous, transformed and/or activated) macrophages that express the CDw52 antigen. Other objects, features and advantages of the present invention will be set forth in the detailed description of preferred embodiments that follows, and in part will be apparent from the description or may be learned by practice of the invention. These objects and advantages of the invention will be realised and attained by the methods and compositions particularly pointed out in the written description and claims hereof.

This object of the present invention is achieved by the use of a ligand of CDw52 for the preparation of a medicament for the treatment of diseases that are related to CDw52 expressing degenerated macrophages. Preferably, the CDw52 expressing degenerated macrophages are selected from the group of MAS-, HLH-, LCH-, and AML-related macrophages.

Surprisingly, it could be found that certain degenerated macrophages express the cellular antigen CDw52, wherein the macrophages are related to the diseases MAS, HLH, LCH, and AML. The effectiveness of inhibitors of the antigen CDw52 for macrophage-related diseases and in particular for inducing apoptosis of macrophages in MAS, HLH, LCH, and AML was a surprising finding. Therefore, the present invention extends the uses of ligands of CDw52 to said cancers and diseases that are currently difficult to treat and/or can not be treated at all.

In a particularly preferred embodiment of the present invention, the treatment occurs by CDw52 mediated apoptosis (cellular death) of the degenerated macrophages. Said "degeneration" means that the macrophages are altered in their physiology in a way that is either caused and/or causing, at least in part, the macrophage-related disease that shall be treated. The degeneration can be selected from transformation (that is, disease-related physiological change of the cell), cancer, hyperproliferation and/or activation.

In principle, the ligand that forms the active principle of the present invention can be selected from all CDw52-interacting ligands, as long as the ligand also leads to the CDw52-mediated death of a CDw52-expressing degenerated macrophage. Thus, according to a preferred aspect of the present invention, the ligand can be selected from a CDw52-specific antibody, a fragment thereof a CDw52-binding peptide and a CDw52-interacting substance.

As already mentioned above, currently CDw52 is already used as a target for therapy in certain leukaemia. Here, the ligand is alemtuzumab (Campath-1H, see above). The leukaemia therapy based on Alemtuzumab is well characterised, and the person of skill in the art will be readily able to amend the described therapeutical regimen in order to treat other CDw52-expressing macrophages. Examples of the respective literature are Moreton P and Hillmen P (Alemtuzumab therapy in B-cell lymphoproliferative disorders. Semin Oncol. 2003 Aug;30(4):493-501. Review.); Cao TM and Coutre SE (Management of advanced chronic lymphocytic leukemia. Curr Hematol Rep. 2003 Jan;2(1):65-72.); Lynn A et al. (Treatment of chronic lymphocytic leukemia with alemtuzumab: a review for nurses. Oncol Nurs Forum. 2003 Jul-Aug;30(4):689-94.); Stull DM (Targeted therapies for the treatment of leukemia. Semin Oncol Nurs. 2003 May;19(2):90-7. Review.); Dearden CE Alemtuzumab in lympho-proliferate disorders. Rev Clin Exp Hematol. 2002 Dec;6(4):435-48; discussion 449-50. Review.); Frampton JE and Wagstaff AJ (Alemtuzumab. Drugs. 2003;63(12):1229-43; discussion 1245-6. Review.); Cao TM and Coutre SE (T-cell prolymphocytic leukemia: update and focus on alemtuzumab (Campath-1H). Hematology. 2003 Feb;8(1):1-6. Review.), and Tall-man MS (Monoclonal antibody therapies in leukemias. Semin Hematol. 2002 Oct;39(4 Suppl 3):12-9. Review.) (all incorporated by reference in their entireties) and the respective literature cited therein. Furthermore, variants of alemtuzumab can also be used for the treatment according to the present invention, as they exhibit improved properties (Hutchins JT, et al. Improved biodistribution, tumor targeting, and reduced immunogenicity in mice with a gamma 4 variant of Campath-1H. Proc Natl Acad Sci U S A. 1995 Dec 19;92(26):11980-4.). CAMPATH-1M is a rat IgM monoclonal antibody which has been used extensively in vitro for purging bone marrow harvests in order to deplete the T cell population prior to bone marrow transplantation. Marked reduction in the incidence and severity of graft-versus-host disease has been seen with this therapy. CAMPATH-1G is a rat IgG2b class-switch variant of an IgG2a antibody recognising the CDw52 antigen which has been used in vivo to achieve immuno-suppression in more than 100 patients undergoing organ and bone marrow transplantation, management of organ rejection and treatment of haematologic malignancies with a high level of success. However, the rapid development of an anti-rat immunoglobulin response, including the possibility of anaphylaxis, is likely to limit the use of rat monoclonal antibodies against the CDw52 antigen in humans in vivo. CAMPATH-1H is a genetically manipulated IgG antibody obtained by grafting the complementarity determining regions from CAMPATH-1G into human framework regions. The resulting "humanised" antibody is highly effective in vitro being equivalent to the rat monoclonal antibody at complement lysis and two to four times better in cell-mediated lysis of human lymphocytes. No limiting anti-globulin response is anticipated with this humanised antibody.

In another particularly preferred embodiment of the present invention, the ligand is administered systemically and/or administered locally. The local administrating has the advantage of reduced side effects by an at the same time increased effective amount of ligand that is present at the site of the macrophage. Subcutaneous administration of alemtuzumab has been described as a key advancement in the treatment of CLL. A phase II trial of subcutaneous alemtuzumab has demonstrated an 87% response rate in 38 previously untreated patients, with a reduction in intravenous administration-related rigors, as well as the elimination of nausea, dyspnea, diarrhoea, and hypotension, frequently seen following intravenous administration of alemtuzumab. Furthermore, encouraging results with the combination of alemtuzumab and fludarabine, which demonstrate eradication of malignant cells in patients who are resistant to either agent alone, were described to open the way for such combinations to produce durable responses even in refractory disease (Rai K, Hallek M. Future prospects for alemtuzumab (MabCampath). Med Oncol. 2002;19 Suppl:S57-63.). This subcutaneous administration of alemtuzumab can be taken as an example to readily apply such route of administration for the treatment of diseases according to the present invention.

The dosage of the ligand of CDw52, preferably the anti-CDw52 antibody, preferably CAMPATH-1H, to be administered to a patient suffering from the present diseases will vary with the precise nature of the condition being treated and the recipient of the treatment. The dose will generally be in the range 1 to about 100 mg for an adult patient, usually administered daily for a period between 1 and 30 days. The preferred daily dose is 1 to 10 mg per day although in some instances larger doses of up to 40 mg per day may be used. Preferably the dosage will be applied in such a manner that the ligand is present in the medicament in concentrations that provide in vivo concentrations of said ligand in a patient to be treated of between 0.01 mg/kg/day and 1 mg/kg/day.

The ligand of CDw52, preferably the anti-CDw52 antibody, particularly CAMPATH-1H will generally be administered to the patient in the form of a pharmaceutical formulation. Such formulations preferably include, in addition to the ligand/antibody, a physiologically acceptable carrier or diluent, possibly in admixture with one or more other agents such as other antibodies or drugs, such as an antibiotic. Suitable carriers include, but are not limited to, physiological saline, phosphate buffered saline, phosphate buffered saline glucose and buffered saline. Alternatively the ligand, e.g. the antibody, may be lyophilised (freeze dried) and reconstituted for use when needed by the addition of an aqueous buffered solution as described above. Routes of administration are routinely parenteral, including intravenous, intramuscular, subcutaneous and intraperitoneal injection or delivery. The administration can be systemic and/or locally.

The ligand of CDw52, particularly the anti-CDw52 antibody, may be administered in combination with or sequentially with a safe and effective amount of other drugs, in particular other drugs conventionally used in the treatment of the above diseases, or other medicaments for the alleviation of specific symptoms of said diseases. Such drug is preferably selected from the group consisting of a DNA-interactive agent, alkylating agent, antimetabolite, tubulin-interactive agent, and a hormonal agent, and is, for example, selected from the group consisting of Methotrexate, Fluorouracil, Fluorodeoxyuridin, CB3717, Azacitidine, Floxuridine, Mercapyopurine, 6-Thioguanine, Pentostatin, Cytarabine, and Fludarabine. Additional chemotherapeutic agents are well-known to the skilled artisan in the field of the therapy of the above diseases. Sequential administration of the ligand/antibody with another drug may be appropriate in some cases (see, for example, Montillo M, et al. Successful CD34+ cell mobilisation by intermediate-dose Ara-C in chronic lymphocytic leukemia patients treated with sequential fludarabine and Campath-1H. Leukemia. 2003 Oct 30).

In another aspect of the present invention, the present invention provides an improved method for screening for CDw52 ligands comprising the steps of: a) incubating a cell expressing CDw52 with a putative ligand, b) measuring, if a binding between CDw52 and said putative ligand occurs, c) in the case of a binding of said ligand to CDw52 is measured, measuring, if said binding between CDw52 and said identified ligand leads to a CDw52-mediated death of a CDw52-expressing solid tumour cell.

In one embodiment, these ligands can be identified based on the structural similarities of CDw52 with other proteins, such as B7-Ag, gp20 or CD24. Examples for ligands of these small glycosylphosphatidylinositol (GPI) anchored peptides are P-selectin and/or glycans as potential recognition signals (Aigner S, et al. CD24, a mucin-type glycoprotein, is a ligand for P-selectin on human tumor cells. Blood. 1997 May 1;89(9):3385-95.; Tone M, et al. Structure and chromosomal location of mouse and human CD52 genes. Biochim Biophys Acta. 1999 Sep 3;1446(3):334-40.).

During the course of the assay of the method according to the invention, the ligand will initially bind or attach to CDw52. The ligand can either bind directly or indirectly to CDw52, i.e. via cofactors that can be present, such as certain ions or protein factors, that promote the attachment of the ligand to CDw52 and therefore support the function of the ligand. "Binding" can occur via a covalent or non-covalent attachment of the ligand or group of ligands to CDw52. Based on the binding properties of the screened ligands, a first pre-selection of ligands can be performed, in which a non-binding ligand is screened in a second "round" of screening using a set of co-factors. If still no binding occurs, the ligand will be classified as "non-binding" and disregarded in further screenings. Such pre-selection will be encompassed by the terms "screening", "measuring" and/or "determining" in the general context of this invention. A ligand that shows an in-vitro modulating (e.g. lytic) action should in vivo preferably not further interact with components of the patients' or test (model) organisms' body, e.g. within the bloodstream, lung and/or heart of the patient or test organism.

In general, assays to determine a binding and biological effect of a ligand to a specific target (in this case CDw52) are well known to the person skilled in the art and can be found, for example, in US patents 4,980,281, 5,266,464 and 5,688,655 to Housey (which are herein incorporated by reference) for phenotypic changes of cells after incubation with a screening ligand. Furthermore, US 5,925,333 to Krieger at al. (which is herein incorporated by reference) describes methods for modulation of lipid uptake and related screening methods.

Suitable tests for showing a biological effect of a ligand for CDw52 include lytic assays that measure the release of intracellular contents (uric acid, potassium, phosphorus) into the extracellular compartment, fluorescence based assays (e.g. use of confocal fluorescent microscopy), viable cell counts, cellular proliferation assays, such as the BrdU proliferation assay or measuring of cellular calcium, and the like.

The method of screening according to the present invention can be performed in several different formats. One embodiment is a method, wherein the assay is *performed in vitro.* The screening assays of the present invention preferably involve the use of macrophage cell-lines (as described below) and other cells, as long as these cells express CDw52. How to produce such recombinant cells is well known to the skilled artisan and is further described in the respective literature.

In another embodiment of the present invention, the ligands are initially screened using an assay such as one of the assays described herein and then tested in, for example, transgenic, CDw52-expressing and/or over-expressing, animals made using standard transgenic animal technology. A technique such as embryonic stem cell technology using rats, mice or hamsters is preferred.

An additional embodiment of the present invention relates to a method wherein the assay is *performed in vivo.* Preferably, the assay is performed in a mouse or rat. In general, *the in vivo* assay will not be substantially different from the above-mentioned in vitro assay. In a general screening assay for ligands of CDw52 will be provided in that the ligand to be tested is/are administered to a mouse or a rat. Then, it will be determined, if said ligand leads to a CDw52-mediated death of a CDw52-expressing macrophage cells compared to the absence of the ligand to be tested, wherein a difference in the death of cells identifies a ligand which leads to a CDw52-mediated death of a CDw52-expressing macrophage cells of said mouse or rat. Of course, these assays can be performed in other non-human mammals as well.

An additional embodiment of the present invention relates to a method according to the invention, wherein said ligand is selected from a library of naturally occurring or synthetic compounds which are randomly tested for binding to CDw52. Such libraries and the methods how to build up such a library, as well as methods for using these libraries for the screening of candidate ligands are well known to the person skilled in the art and further described in the respective literature. Furthermore, some of these libraries are commercially available. The present invention contemplates high throughput screening of ligands for CDw52. The ligands as described above, and modifications of said ligands, including analogues, derivatives, fragments, active moieties, and the like, may be screened using methods and systems of the present invention.

In the context of the present invention, a "CDw52-mediated death" encompasses all biological effects within a cell that are mediated an/or initiated by a binding of a ligand of CD52 and lead to a negative effect for the viability and/or proliferation of the cell or tissue that expresses CDw52, in particular macrophages. Without limitation, such negative effects include the lysis of the cell, apoptosis and/or inhibition of the proliferative activity of the cell. Preferably, the effect is the CDw52-mediated apoptosis of the cell (for references, see above).

An additional embodiment of the present invention relates to a method for the production of a pharmaceutical formulation, comprising the steps of: a) performing a method as given above, and b) formulating the identified ligand for CDw52 with pharmaceutically acceptable carriers and/or excipients. Such formulations therefore include, in addition to the ligand/antibody, a physiologically acceptable carrier or diluent, possibly in admixture with one or more other agents such as other antibodies or drugs, such as an antibiotic. Suitable carriers include, but are not limited to, physiological saline, phosphate buffered saline, phosphate buffered saline glucose and buffered saline. Alternatively the ligand, e.g. the antibody, may be lyophilised (freeze dried) and reconstituted for use when needed by the addition of an aqueous buffered solution as described above. Routes of administration are routinely parenteral, including intravenous, intramuscular, subcutaneous and intraperitoneal injection or delivery. The administration can be systemic and/or locally.

Finally, the present invention provides novel methods for the treatment of macrophage-related diseases and in particular MAS, HLH, LCH and/or AML with macrophages that express CDw52 by the application to a patient in need thereof of an effective amount of a ligand, in particular an inhibitor, of the antigen CDw52 in order to induce the CDw52-mediated cellular death of the macrophage cell that relate to the disease to be treated. The major advantages of this novel therapy are its application in areas of the body where a surgical intervention is impossible, in the case of a multifocal disease statuses, and the application in proliferating cancers where current chemotherapeutical approaches can not be employed or are not effective. Furthermore, the spectrum of side effects of the treatment based o CDw52 is regarded as low (Lockwood CM, Hale G, Waldman H, Jayne DR. Remission induction in Behcet's disease following lymphocyte depletion by the anti-CD52 antibody CAMPATH 1-H. Rheumatology (Oxford). 2003 Aug 29). Finally, the follow-up use of the ligands for CDw52 after surgery or other treatment in order to ameliorate the disease is envisaged in order to further minimise the size of the residual tumour (if not fully removed) and/or to avoid relapses.

The present invention provides a method of treatment of a human subject suffering from a macrophage-related disease, wherein the macrophage expresses the CD52 antigen, such as MAS, HLH, LCH and/or AML, which comprises administering to the said subject an effective amount of an antibody recognising the CDw52 antigen. Suitable formulations, routes of administrations and dosages are indicated above and are further laid out in the following examples. Further examples are described in, for example, Keating MJ, et al. (Campath-1H treatment of T-cell prolymphocytic leukaemia in patients for whom at least one prior chemotherapy regimen has failed.J Clin Oncol. 2002 Jan 1;20(1):205-13.); Montillo M, et al. (Safety and efficacy of subcutaneous Campath-1H for treating residual disease in patients with chronic lymphocytic leukemia responding to fludarabine. Haematologica. 2002 Jul;87(7):695-700).

In summary, the finding of the present invention that CDw52 is expressed in certain disease-related macrophages, such as macrophages related to MAS, HLH, LCH and/or AML, will form the basis for the extension of an disease therapy based on ligands of CDw52 and in particular of CDw52-specific antibodies such as alemtuzumab (Campath-1H). The major advantages of this novel therapy are its application in areas of the body where a surgical intervention is impossible, in the case of a multifocal disease status and the application in slowly proliferating cancers, where the current chemotherapeutical approaches can not be employed.

"Pharmaceutical composition" shall mean a composition comprising at least one active ingredient, whereby the composition is amenable to investigation for a specified, efficacious outcome in a mammal (for example, and not limitation, a human). Those of ordinary skill in the art will understand and appreciate the techniques appropriate for determining whether an active ingredient has a desired efficacious outcome based upon the needs of the artisan, in this case an anti-cancer therapy based on ligands of CDw52.

"Treatment" or "therapy" shall mean a process of the reversing or ameliorating or reducing a condition of a certain type or certain types of cells or tissues with respect to an abnormal behaviour, such as a macrophage-related disease, such as MAS, HLH, LCH and/or AML. Preferably, a treatment is applied to a patient.

A "fragment" of a ligand, in particular a fragment of an antibody, shall mean a moiety that is derived from the ligand that is still capable of binding to the respective cellular marker (for example, CDw52). Particular examples for antibodies are scFV-fragments and other antibody-derived peptides that can bind to the respective cellular marker. In a preferred embodiment, the binding of the fragment leads to the same biological effect(s) as the binding of the full-length (or sized) ligand.

The present invention shall now be further described based on the following examples without being limited thereto with reference to the accompanying figures, in which

Figure 1 shows the results of the ⁵¹Cr release assay for the macrophage-cell line THP-1. The lysis (apoptosis) is at approximately 70% after 18h.

Figure 2 shows the results of the ⁵¹Cr release assay for the macrophage-cell line U-937. The lysis (apoptosis) is at approximately 55% after 18h.

### Examples

### Immunohistochemical analysis of the CDw52 expression in macrophage-derived cell lines

For the immunohistochemical analysis of CDw52 on macrophages using the doubled APPAP-method of the commercially available Dako-Production kits, 1 µm thickness paraffin sections were made on poly-L-Lysin coated object slides (OS). The slides were incubated over night at 58°C. On the next day, the de-paraffination occurs by means of a serial alcohol treatment, wherein the following steps were performed: 10 minutes xylol, 5 minutes xylol, two times 5 minutes alcohol abs., 2 times 5 minutes 96 % alcohol, two times 5 minutes 70 % alcohol and subsequently 2 times 5 minutes aqua dest. After this treatment, the OS are put in PBS-2 % BSA (rinsing buffer). A proteolytic treatment with proteinase K for 5 minutes is performed. Subsequently, the incubation of the primary antibody (rat anti human, Serotec MCA 1642, 1:40) is performed for 30 minutes with subsequent rinsing for two times for 3 minutes in the buffer. The identical time periods are used for the secondary antibody (rabbit anti rat, Dako Z 0455, 1:50) and the APPAP-complex (rat, Dako D 0488, 1:100), wherein after each step, a rinsing for two times with buffer for 3 minutes is done. Due to the fact that this method is the doubled APPAP, another incubation for 10 minutes is performed using first the secondary antibody and subsequently the APPAP-complex. After rinsing for two times for 3 minutes, the chromogen is added (Fuchsin⁺, Dako K 0625) for 10 minutes which is washed away with water after the incubation period. The staining of the nucleus occurs for 5 minutes with hematoxylin. Subsequently, the nucleus is blue-dyed for 5 minutes and the OS covered with Aquatex.

In an immunohistochemical analysis of the CDw52 expression in macrophage cell lines, the expression of CDw52 could be verified.

### RNA Isolation and RT-PCR analysis

RNA was isolated from frozen samples using the RNeasy-mini-kit (Qiagen). For RT-PCR a one-step RT-PCR kit (Cat. No. 210210) from Qiagen was used. All primer sequences and the annealing temperatures for RT-PCR for each of the genes are depicted in **Table 1.** β-Actin was amplified together with each RT-PCR reaction to ensure cDNA integrity.

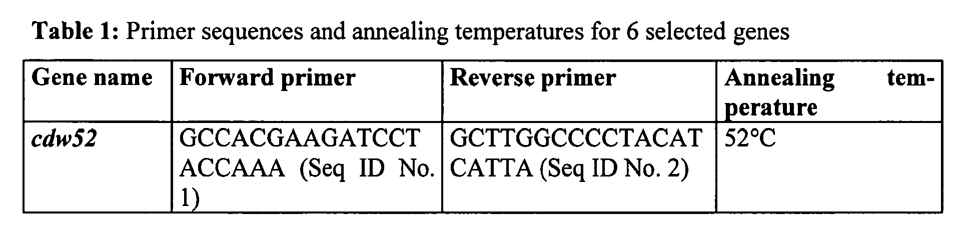

### Antibody-mediated cellular cytotoxicity of CDw52 (ADCC, ⁵¹Cr release assay)

A ⁵¹Cr release assay was performed with antibodies of CDw52 using the commonly available macrophage-cell lines THP-1 and U-937 (received from DSMZ (German Collection of Microorganisms and Cell Cultures) in Braunschweig, Germany). For the assay, the following protocol (according to Rebello P and Hale G 2002, slightly modified) was used.
- Draw 30 ml blood from the patient to be analysed
- Ficoll-resolution of the LAK-cells by gradient-centrifugation
- 30 ml blood + 30 ml 1xPBS
- 15 ml Ficoll-solution in 50 ml tubes; add PBS/blood
- centrifuge 10 min 500xg
- remove lymphocytes (LAK-cells) using pipette (turbid phase) and transfer into new tube
- wash 2 x 5 min 1000 rpm (1 ml with 1xPBS, 1 x with medium)
- count cells
- add 2000 IU/10 ml IL-2
- incubate 72 h at 37°C
- determine cell-count of the cells to be examined
- Centrifuge 5 min 1000 rpm at 4°C
- resuspend cells in 10 ml medium
- 1*10⁶ cells/1,5 ml; label with 0,5mCi ⁵¹Cr
- incubate 2 h 37°C (shake every 30 min)
- prepare PAK 1:100 (sterile)
- prepare LAK-cells (cells were counted and diluted to concentrations 1:2,5 - 1:5 ect.)
- fill plates with all samples (200µl per well, 100µl Target-cells, 75µl LAK-cells (only at E:T-ratio), 25µl CAMPATH, 10µl SDS (at maximum release), add to 200µl)
- Centrifuge plate at 1000 rpm 10 min
- put 80 µl of supernatant into tubes
- measure ⁵¹Cr in supernatant
- after 18 h, centrifuge again at 1000 rpm for 10 min
- put 80 µl of supernatant into tubes
- measure ⁵¹Cr in supernatant

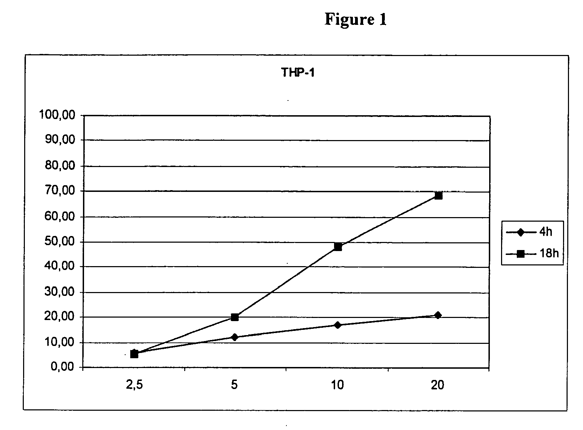

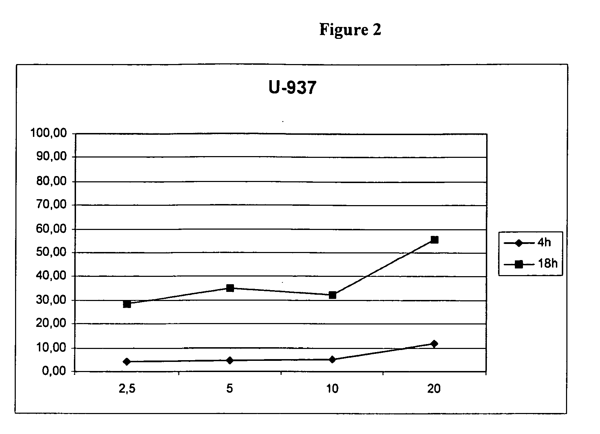

Figure 1 shows the results of the ⁵¹Cr release assay for the macrophage-cell line THP-1. The lysis (apoptosis) is at approximately 70% after 18h. Figure 2 shows the results of the ⁵¹Cr release assay for the macrophage-cell line U-937. The lysis (apoptosis) is at approximately 55% after 18h..

### Treatment of a macrophage related disease, such as MAS, HLH, LCH or AML based on a CDw52 specific antibody

### Treatment scheme A):

The patients (optionally including chemotherapy-naive patients) will receive 3, 10, and 30 mg of Campath-1H on sequential days, followed by 30 mg three times weekly, as 2-hour intravenous infusions, for 4 to 12 weeks. The objective response rate will be expected at about 50% (40% to 63%), with an about 40% complete response (CR) rate (28% to 51%). The most common Campath-1H-related adverse events will be expected as acute reactions during or immediately after infusions. Infectious episodes during treatment may lead to treatment discontinuation.

### Treatment scheme B):

As intravenous Campath-1H is almost invariably associated with reactions, sometimes of WHO grade 3-4, the subcutaneous route of administration will be adopted, which proves to induce rare and mild adverse reactions but usually has comparable efficacy.

The patients who, optionally, responded to FAMP will received subcutaneous Campath-1H, three times a week for 6 weeks in escalating doses up to 10 mg. The patients will receive acy-clovir and cotrimoxazole as infection prophylaxis. Granulocyte colony-stimulating factor (G-CSF), at the dosage of 5-10 microg/kg/day, or intermediate-dose Ara-C (800 mg/m(2)/q 12h x 6 doses), can be administered to obtain peripheral blood stem cell (PBSC) mobilisation.

### Treatment scheme C):

The patient will received treatment with CAMPATH-1H (2 mg/day iv for five days, two days rest, then 10 mg/day iv for five days). The "double-peaked" pattern will be seen with neutrophils which increases during the same time course and coincides with the second and third dosing days at 2 and 10 mg respectively (10 mg doses will follow a two day rest).

The 10 dose CAMPATH-1H treatment course should be well tolerated. Fever and headache might be reported as adverse events with both occurring with administration of the first 2 mg and 10 mg doses.

The person of skill will be readily able to amend the above given treatment schemes with respect to other ligands, such as protein, in particular protein or peptide mimetica of anti-CDw52 antibodies, glycans and P-selectin.

## Claims

1. Use of a ligand of CDw52 for the preparation of a medicament for the treatment of diseases that are related to CDw52 expressing degenerated macrophages.

2. Use according to claim 1, wherein the CDw52 expressing degenerated macrophages are selected from the group of MAS-, HLH-, LCH-, and AML-related macrophages.

3. Use according to claim 1 or 2, wherein the treatment occurs by CDw52 mediated apoptosis of the degenerated macrophages.

4. Use according to any of claims 1 to 3, wherein the degeneration is selected from transformation, cancer, hyperproliferation and/or activation.

5. Use according to any of claims 1 to 4, wherein the ligand is selected from a CDw52-specific antibody, a fragment thereof a CDw52-binding peptide and a CDw52-interacting substance.

6. Use according to claim 5, wherein the ligand is alemtuzumab (Campath-1H).

7. Use according to any of claims 1 to 6, wherein the ligand is administered systemically and/or administered locally.

8. Use according to any of claims 1 to 7, wherein the ligand is present in the medicament in concentrations that provide in vivo concentrations of said ligand in a patient to be treated of between 0.01 mg/kg/day and 1 mg/kg/day.

9. Use according to any of claims 1 to 8, wherein the ligand is for administration in combination with other chemotherapeutically active substances.

10. A method for screening for CDw52 ligands comprising the steps of:
a) incubating a cell expressing CDw52 with a putative ligand,
b) measuring, if a binding between CDw52 and said putative ligand occurs,
c) in the case of a binding of said ligand to CDw52 is measured, measuring, if said binding between CDw52 and said identified ligand also leads to a CDw52-mediated death of a CDw52-expressing macrophage cell.

11. Method for the production of a pharmaceutical formulation, comprising the steps of:
a) performing a method according to claim 10, and
b) formulating the identified ligand for CDw52 with pharmaceutically acceptable carriers and/or excipients.
